Europäisches Patentamt

(19) European Patent Office    (11) Publication number: **0 011 161**
Office européen des brevets    **B1**

(12) **EUROPEAN PATENT SPECIFICATION**

(45) Date of publication of patent specification: **14.10.81**    (51) Int. Cl.³: **C 07 G 7/00,** C 08 H 1/00, //A 61 K 7/02, A 61 K 37/12

(21) Application number: **79104123.9**

(22) Date of filing: **24.10.79**

(54) Finely powdered fibroin and process for producing same.

| | |
|---|---|
| (30) Priority: **13.11.78 JP 139973/78** **17.04.79 JP 47382/79** | (73) Proprietor: **KANEBO LTD.** **17-4 sumida 5-chome sumida-ku** **Tokyo (JP)** Proprietor: **Kanebo Spun Silk, Ltd.** **1623 Nakamaruko Maruko-machi** **Chiisagata-gun, Nagano-ken (JP)** |
| (43) Date of publication of application: **28.05.80 Bulletin 80/11** | |
| (45) Publication of the grant of the European patent: **14.10.81 Bulletin 81/41** | (72) Inventor: **Ohtomo, Koichiro** **26-1 Higashiyosumi-cho 1-chome** **Takatsuki** **Osaka-fu (JP)** Inventor: **Korikawa, Yokio** **18-5, Aho 3-chome, Matsubara** **Osaka-fu (JP)** Inventor: **Otoi, Kiyoshi** **1623 Nakamaruko Maruko-machi** **Chiisagata-gun Nagano-ken (JP)** |
| (84) Designated Contracting States: **DE FR GB** | |
| (56) References cited: Chemical Abstracts vol. 70, no. 24, 1969 Columbus, Ohio, USA K. HIRABAYASHI et al. "Crystallization of regenerated silk fibroin (Bombyx mori) from its aqueous solution" page 48, column 1, abstract no. 107 376z | (74) Representative: **Eitle, Werner, Dipl.Ing., et al** **Arabellastrasse 4 Sternhaus** **D-8000 München 81 (DE)** |
| Chemical Abstracts vol. 87, no. 14, 1977 Columbus, Ohio, USA Y. ISHIGURO et al. "Conformational change of powdered silk fibroin" page 44, column 2, abstract no. 103 275u | |

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

## Finely Powdered Fibroin and Process for Producing Same

This invention relates to finely powdered high-purity fibroin and a process for producing the same.

Powdered silk fibroin is considered to be useful as an additive for cosmetic and pharmaceutical preparations, because of its moderate moisture absorption and retention properties and its high affinity for the human skin. Currently available silk fibroin powders are generally produced by finely dividing silk thread with a pulverizer. Such a silk fibroin powder consists of filamentous fibers cut in very short lengths rather than nearly globular particles and, when used as an additive for cosmetic and pharmaceutical preparations, gives rise to various difficulties. For example, in mixing the powder with other ingredients in globular form, it is so liable to aggregation that a homogeneous final product is hardly obtained. Even if such a product is obtained, it shows poor slip properties upon application to the human skin and may occasionally produce round agglomerates of silk fibroin. Thus, it can be said that these difficulties prevent us from making good use of excellent properties of silk fibroin.

With the background the present inventors have made repeated studies on the production of a homogeneous fine powder of fibroin in globular particulate form rather than in fibrous form. In this field of art, for example, a process for producing silk fibroin suitable for use in chromatography is disclosed in Japanese Patent Publication No. 1941/'64. This process comprises dissolving silk fibroin in a cupr-ammonium solution or a solution of a copper complex (for example, a cupri-ethylenediamine solution), neutralizing the resulting solution with an acid, and then adding an alcohol to the neutralized solution to form a white precipitate of silk fibroin. As a result of confirmatory tests made by the present inventors, it has been found that this process requires a very large amount of alcohol and, moreover, the resulting precipitate is too sticky to be separated by filtration. Another process for producing a powder of silk fibroin is disclosed in Japanese Patent Publication No. 4947/'51. This process comprises dissolving degummed silk fiber in a concentrated aqueous solution of a neutral salt such as calcium nitrate, dialyzing the resulting solution, and spraydrying the colloidal solution so formed. However, the powder of silk fibroin thus obtained is abnormally hydrophilic and, therefore, unsuitable for use as an additive for cosmetic preparations.

In addition, there have been proposed other processes which involves hydrolyzing silk thread with an acid or alkali to prepare a silk fibroin solution and then precipitating the silk fibroin either by neutralization or by the addition of an alcohol. In the fine powders of silk fibroin produced by these processes, however, the molecular weight is reduced to those of oligomers and the characteristic properties of silk are completely lost.

According to "Seni Gakkaishi, Vol. 24, Nor. 9 (1968) pp. 449—450" referred to in Chemical Abstract 70, 107 376z fibroin films are made from regenerated fibroin solutions which were prepared by dissolving silk in a concentrated lithium bromide solution. From this solution films were prepared showing $\alpha$- and $\beta$- forms of fibroin. According to "Semi Gakkaishi, Vol. 24, No. 9 (1968), pp. 449—450" referred to in Chemical Abstract 70, 107376z, fibroin is prepared by pulverizing the fibroin of $\alpha$-configuration obtained by extracting the degummed silk with ethyl ether. The resulting fibroin is a product which is formed by pulverizing the particles of fibroin included in the degummed silk. Because the degummed silk contains the fibroin in the fibrilled state a fibrous fine powder is obtained by the pulverization, which is undesirable as additive for cosmetics and medical preparations.

X-ray diffraction, analysis and infrared spectroscopic analysis have revealed that, in the conventional fine powders of silk fibroin produced by various processes involving the dissolution of silk fibroin, the silk fibroin molecules contained therein have either a random configuration or the $\alpha$-configuration and the degree of crystallinity is so low as to imply the amorphous state rather than the crystalline state.

In order to overcome the above described difficulties, the present inventors have made a series of intensive and extensive studies on the principle of rendering finely powdered fibroin hydrophobic to such a degree that it shows no stickiness in the presence of water, and have thereby completed this invention.

It is an object of this invention to provide a fine powder of fibroin in nonfibrous and particulate form. Another object of this invention is to provide a fine powder of fibroin in nonfibrous and particulate form which, when used as an additive for cosmetic preparations, exhibits good compatibility and dispersibility with other base materials, moderate swelling properties, and excellent slip properties without adherence to the skin. Still another object of this invention is to provide a process for producing a fine powder of fibroin in nonfibrous and particulate form which permits its industrial production with great ease and at low cost.

In accordance with one aspect of this invention, there is provided a regenerated fibroin in nonfibrous and particulate form which has an average molecular weight of not less than 50,000, particle diameters of from 1 to $100\mu$, and a bulk density of from 0.1 to $0.7$ g/cm$^3$ as measured in the dry state and which contains at least 50% by weight of hot-

water insoluble fibroin having the $\beta$-configuration.

In accordance with another aspect of this invention, there is provided a process for producing such a fine powder of fibroin in nonfibrous and particulate form. In one specific embodiment, the process comprises the steps of dissolving a degummed silk material in at least one solvent selected from the group consisting of an aqueous cupri-ethylenediamine solution, an aqueous ammoniacal solution of cupric hydroxide, an aqueous alkaline solution of cupric hydroxide and glycerol, an aqueous lithium bromide solution, an aqueous solution of the chloride, nitrate of thiocyanate of calcium, magnesium or zinc, and an aqueous sodium thiocyanate solution; adding a coagulating salt to the resulting aqueous fibroin solution having a fibroin concentration of from 3 to 20% by weight to coagulate and precipitate the fibroin; dehydrating and drying the gel so formed; and then pulverizing the resulting powder. In another specific embodiment, the process comprises the steps of dissolving a degummed silk material in at least one solvent selected from the group consisting of an aqueous cupriethylenediamine solution, an aqueous ammoniacal solution of cupric hydroxide, an aqueous alkaline solution of cupric hydroxide and glycerol, an aqueous lithium bromide solution, an aqueous solution of the chloride, nitrate or thiocyanate of calcium, magnesium or zinc, and an aqueous sodium thiocyanate solution; dialyzing the resulting aqueous fibroin solution; subjecting the dialyzed aqueous fibroin solution having a fibroin concentration of from 3 to 20% by weight to at least one treatment for coagulating and precipitating the fibroin, the treatment being selected from the group consisting of the addition of an alcohol, the addition of a coagulating salt, aeration, coagulation at the isoelectric point, exposure to ultrasonic waves, and agitation at high shear rate; dehydrating and drying the gel so formed; and then pulverizing the resulting powder

The fine powder of fibroin in nonfibrous and particulate form which is produced by the process of this invention has an average molecular weight of not less than 50,000, a degree of molecular orientation equal to not greater than one-half that of natural silk thread, particle diameters of from 1 to 100 $\mu$, and a bulk density of from 0.1 to 0.7 g/cm$^3$, and contains at least 50% by weight of hot water insoluble fibroin having the $\beta$-configuration.

The solvent for fibroin which is used in the process of this invention can be an aqueous cupri-ethylenediamine solution, an aqueous ammoniacal solution of cupric hydroxide (Schweitzer's reagent), an aqueous alkaline solution of cupric hydroxide and glycerol (Roe's reagent), an aqueous lithium bromide solution, an aqueous solution of the chloride, nitrate, or thiocyanate of calcium, magnesium, or zinc, and an aqueous sodium thiocyanate solution.

However, it is preferable to use an aqueous solution of the chloride or nitrate of calcium or magnesium, because of its low cost and convenience for use. The concentrations of these aqueous solutions may vary according to the type of solute used, temperature, and the like. Where an aqueous solution of a metal salt is used, its concentration is generally from 5 to 80% by weight, preferably from 20 to 70% by weight, and most preferably 25 to 60% by weight.

The silk material which is used in the process of this invention can be cocoons, raw silk, waste cocoons, raw silk waste, bisu (unreelable cocoons), silk fabric waste bourette, and the like. Prior to use, the silk material is degummed or freed from sericin by any conventional procedure. For example, it is washed in warm water containing a surface active agent or an enzyme according to the need, and then dried.

Using a suitable apparatus such as kneader, the degummed silk material is dissolved in the solvent which is selected from among the aforesaid aqueous solutions and preheated to a temperature of from 60 to 95°C and preferably from 70 to 85°C. The solvent is generally used in an amount of from 2 to 50 parts by weight and preferably from 3 to 30 parts by weight per parts by weight of the degummed silk material.

Where the coagulation step is carried out by the addition of a coagulating salt, the resulting aqueous fibroin solution may be used directly. However, the aqueous fibroin solution must be dialyzed before it can be subjected to any one of the other treatments. It is also preferable to dialyze the aqueous fibroin solution in case of the addition of a coagulating salt.

In the dialysis step, the salt contained in the aqueous silk fibroin solution is almost completely removed by means of a dialyzer using semipermeable membranes or hollow fibres, typically made of cellophane. In order that a gel of fibroin may be formed stably and rapidly, there must be a proper corrolation between the volume of the solution to be dialyzed and the surface area of the dialysis membrane. More specifically, desalting should be carried out by the use of a multilayer membrane structure or bundled hollow-fiber structure satisfying the condition expressed by

$$\frac{\text{Membrane Surface Area (cm}^2)}{\text{Priming Volume (cm}^3)} \geq 10$$

where the priming volume means the internal volume within the tubing or between the layers. If the value of the above-defined ratio is less than 10, the removal of the salt through the membrane is not effected rapidly and, moreover, not a stable gel of fibroin but only a sticky precipitate is formed in the succeeding coagulation step. In order to to carry out the process of this invention smoothly and economically, the above-defined ratio should preferably have a value of not less than 30 and most preferably a value of not less than 50. In the case of a multilayer membrane structure, for example, it is necessary to keep the spacing between layers at 2 mm or less for the purpose of satisfying the aforesaid condition. In the case of a bundled hollow-fiber structure which is more suited to the satisfaction of the aforesaid condition, it is necessary to use hollow fibers having a diameter of 4 mm or less.

In the process of this invention, the dialyzed aqueous fibroin solution has very low residual salt concentration of from 0.003 to 0.06% by weight, so that an extremely high purity of fibroin can be achieved.

Before being transferred to the coagulation step, the aqueous fibroin solution is adjusted to a fibroin concentration of from 3 to 20% by weight, perferably from 4 to 15% by weight, and most preferably from 5 to 10% by weight. Where the dialysis step is omitted, the aqueous fibroin solution is necessarily prepared so that it will have a fibroin concentration of from 3 to 20% by weight. Where the aqueous fibroin solution is subjected to dialysis, however, the dialyzed aqueous fibroin solution may be either concentrated or diluted until the desired fibroin concentration is attained. If the fibroin concentration is less than 3% by weight, a homogeneous mass of gel is not formed and a long time is required for the coagulation step (which leads to an economic loss), while if it is greater than 20% by weight, a stable mass of gel is formed but its dehydration becomes very difficult.

After adjustment of the fibroin concentration, the aqueous fibroin solution is subjected to the coagulation step. First of all, a homogeneous mass of gel can be formed by the addition of an alcohol to the aqueous fibroin solution having an appropriate fibroin concentration within the above-defined range. The amount of alcohol added should generally be from 1 to 150% by weight, preferably from 5 to 80% by weight, and most preferably from 10 to 60% by weight based on the weight of the aqueous fibroin solution. If the amount of alcohol added is less than 1% by weight, no gel is formed and, even if it is formed, a very long time is required. On the other hand, if it is greater than 150% by weight, a sticky precipitate rather than a stable mass of gel is formed and its dehydration becomes very difficult. There is a correlation between the amount of alcohol added and the fibroin concentration of the aqueous fibroin solution. More specifically, the product of the fibroin concentration (% by weight) and the amount of alcohol added (% by weight) should generally have a value of from 10 to 10,000 and preferably from 15 to 500. The alcohol may be added to the aqueous fibroin solution, and vice versa. In either case, a homogeneous and stable mass of gel is usually formed in an instant or within several hours.

The alcohols which can be used in the process of this invention include monohydric aliphatic alcohols such as methyl alcohol, ethyl alcohol, propyl alcohol, isopropyl alcohol, butyl alcohol, pentyl alcohol, octyl alcohol, etc.; dihydric aliphatic alcohols such as ethylene glycol, diethylene glycol, triethylene glycol, etc.; and trihydric alcohols such as glycerol. However, methyl alcohol, ethyl alcohol, and isopropyl alcohol are preferred because they permit a stable mass of gel to be formed easily.

Where the addition of a coagulating salt is used, a concentrated aqueous solution of a salt such as sodium chloride, potassium chloride, sodium sulfate, potassium sulfate, ammonium sulfate, sodium nitrate, potassium nitrate, etc. is added to and mixed with the aqueous fibroin solution and the resulting mixture is stirred to precipitate the fibroin. (If a calcium salt is used in the dissolution step and a sulfate in the coagulation step, a coprecipitation of calcium sulfate takes place.) The concentration of the concentrated aqueous solution of a coagulating salt is usually adjusted so that the resulting mixture will contain from 5 to 10% by weight of the coagulating salt.

Aeration is carried out by bubbling air through the aqueous fibroin solution according to any suitable technique. For each liter of the aqueous fibroin solution, air is usually fed at a rate of at least 0.1 l-min. The aeration time is generally 10 minutes or more, though it depends on the feed rate of air.

Coagulation at the isoelectric point is carried out by adding an inorganic acid (such as hydrochloric acid, sulfuric acid, etc.) or an organic acid (such as acetic acid, citric acid, etc.), with stirring, to the aqueous fibroin solution until its pH reaches 4.5. Then, this aqueous fibroin solution is generally allowed to stand at room temperature for a period of 10 minutes or more.

Exposure to ultrasonic waves is carried out by placing the aqueous fibroin solution in an ultrasonic wave generator and exposing it, with stirring, to ultrasonic waves which generally have frequencies of 30 KHz or higher. The fibroin is coagulated by continuing this treatment at room temperature for a period of 1 hour or more.

The fibroin can also be precipitated simply by agitating the aqueous fibroin solution. However, this agitation must be carried out at a high shear rate which is generally 50/sec or more and preferably 100/sec or more. The agitation time required for gelation is generally 1 hour or more, though it depends on the concentration of the aqueous fibroin solution, the shear rate, and the like.

During this agitation, methyl alcohol, ethyl alcohol, isopropyl alcohol, or acetone may be added to the aqueous fibroin solution so that the rate of $\beta$-configuration can be raised to the order of 70%. The amount of alcohol or acetone added is suitably from 1 to 100% by weight based on the weight of the aqueous fibroin solution.

The mass of gel so formed is subjected to the dehydration step. This step is preferably carried out by the use of a centrifuge, and the stable mass of gel formed in accordance with this invention is generally dehydrated to a water content of the order of from 100 to 500% by weight based on the weight of the solid contained therein. During the dehydration step carried out by centrifugation, the mass of gel is broken to fragments of small sizes which can then be easily dried to the absolute dry state. This drying step is carried out at a temperature of from 60 to 120°C under normal or reduced pressure.

The powder of fibroin thus obtained is subsequently pulverized by the use of a pulverizer such as hammer mill or jet mill. The particle diameters should be adjusted to a range of from 1 to $100\mu$, preferably from 4 to $80\mu$ and most preferably from 5 to $30\mu$. If the particle diameters are less than $1\mu$, the resulting fine powder shows poor dispersibility and compatibility when used as an additive for cosmetic preparations, while if they are greater than $100\mu$, the resulting fine powder has low affinity for the skin and poor slip properties on the skin. Since the present process for producing a fine powder of fibroin involves gelation followed by dehydration and drying, the resulting fibroin particles are considered to have very minute pores to which their good moisture absorption and retention properties are attributable. However, this may lead to the disadvantage that the fine powder of fibroin becomes excessively swollen in certain applications. It is desirable, therefore, to subject the resulting fine powder of fibroin to a wet heat treatment comprising exposure to saturated steam at a temperature of 50°C or above and preferably from 80 to 120°C. This treatment may be applied either to the powder ensuing from the dehydration and drying step or to the fine powder ensuing from the pulverization step. Moreover, the fine powder of fibroin can further be insolubilized in hot water by, prior to drying, heating the dehydrated gel at a temperature of 50°C or above in an aqueous solution of a neutral salt such as sodium chloride, potassium chloride, sodium sulfate, potassium sulfate, ammonium sulfate, sodium nitrate, etc. or in an organic solvent such as acetone, alcohol, etc.

The fine powder of fibroin produced in accordance with this invention has a degree of crystallinity of not less than 20%, preferably not less than 30%, and most preferably not less than 40%. The degree of crystallinity was determined in the following manner: An aqueous fibroin solution containing 5% by weight of a fibroin produced in accordance with this invention was poured on a Teflon plate and then dried at a temperature of 50°C to form a fibroin film having a thickness of about $60\mu$. This fibroin film was regarded as amorphous (0%) while raw silk as completely crystalline (100%). The degree of crystallinity was expressed as a relative value on the scale defined between these standard points.

The fine powder of fibroin produced in accordance with this invention has a bulk density of from 0.1 to 0.7 $g/cm^3$ and preferably from 0.2 to 0.6 $g/cm^3$ as measured in the dry state. If the bulk density is less than 0.1 $g/cm^3$, the fine powder of fibroin has poor compatibility and dispersibility and, when used as an additive for cosmetic preparations, may produce a phase separation. If it is greater than 0.7 $g/cm^3$, the fine powder of fibroin is decreased in moisture absorption and retention properties. The bulk density was measured in the most closely packed state by means of a commercially available powder tester (manufactured and sold by Hosokawa Tekkosho, Ltd.).

The fine powder of fibroin produced in accordance with this invention contains at least 5.0% by weight of hot-water-insoluble fibroin having $\beta$-configuration. If the content of hot-water-insoluble fibroin is less than 50% by weight, the fine powder is extremely hydrophilic and liable to deterioration. Moreover, when used as a base material for cosmetic preparations, it shows a high degree of stickiness and gives a disagreeable feeling to the skin. The content of hot-water-insoluble fibroin (or rate of $\beta$-configuration was determined in the following manner: 10 g (absolute dry weight) of a fine powder of fibroin to be tested was boiled in 1 l of hot water at a temperature of 100°C for a period of 15 minutes, and the undissolved fraction of fibroin was absolutely dried and weighed. Then, the content of hot-water-insoluble fibroin was calculated from the equation:

Content of Hot-water-insoluble Fibroin

$$= \frac{W}{10} \times 100 \text{ (\% by weight)}$$

where W stands for the absolute dry weight (in g) of the undissolved fraction of fibroin.

The fine powder of fibroin produced in accordance with this invention has a high purity as well as good moisture absorption and retention properties. Accordingly, it is very useful as an additive for cosmetic and pharmaceutical preparations. It is also suitable for use as an adsorbent in pharmaceutical and hygienic applications, because the fibroin particles contained therein have very minute pores owing to the special manner of production.

The present invention is further illustrated by the following examples.

Example 1

In this example, raw silk waste was used as the starting material for the production of fine powders of fibroin. Three kg of raw silk waste was immersed in 100 l of water containing 0.3% by weight of marseille soap, stirred at 80°C for 1 hour, treated with a sericinolytic enzyme to remove the sericin almost completely, washed with water, and then dried.

By stirring in a kneader, 1-kg portions of raw internal diameter of 200$\mu$, a membrane thickness of 20$\mu$, and a length of 500 mm, both ends of these hollow fibers being bundled and sealed silk waste degummed as above were dissolved in 10 kg each of aqueous calcium chloride solutions having the respective calcium chloride concentrations indicated in Table 1. As can be seen from the data of this table, the process of this invention allowed the raw silk waste to be dissolved easily. However, when the calcium chloride concentration was less than 5% by weight as in Control Runs 1-(1) and 1-(2), it was hardly dissolved even after a long period of time (24 hours or more).

The resulting aqueous fibroin solutions were then desalted by passing each of them through a dialyzer of the hollowfiber type at a rate of 1 l/hr. This dialyzer was composed of 2,000 hollow fibers of regenerated cellulose having an without blocking up their hollow bores. In this case, the ratio of membrane surface area (cm$^2$) to priming volume (cm$^3$) had a value of 100. After completion of the dialysis, the aqueous fibroin solutions had fibroin concentrations of 5.3—6.7% by weight and residual calcium chloride concentrations of 0.007—0.033% by weight.

The molecular weight of the fibroin contained in each of the dialyzed aqueous fibroin solutions was measured by gel permeation chromatography. When the calcium chloride concentration was greater than 80% by weight as in Control Run 1-(15), the molecular weight was reduced to the order of 40,000. In the aqueous fibroin solutions prepared in accordance with this invention, however, the fibroin contained therein had a molecular weight of not less than 50,000 and showed no appreciable degree of hydrolysis.

TABLE 1

| | Calcium Chloride Concentration* (wt. %) | Amount of Ethyl Alcohol Added** (wt. %) | Temperature (°C) | Time (hr) | Solubility *** | Molecular Weight ($\times 10^4$) |
|---|---|---|---|---|---|---|
| 1—(1) Control Run | 3 | 0 | 95 | 24 | X | — |
| 1—(2) ,, | 3 | 30 | 85 | 24 | X | — |
| 1—(3) Test Run | 5 | 30 | 85 | 5 | Δ | 8 |
| 1—(4) ,, | 10 | 30 | 80 | 5 | Δ | 9 |
| 1—(5) ,, | 30 | 30 | 80 | 2 | O | 9 |
| 1—(6) ,, | 40 | 0 | 95 | 2 | O | 7.5 |
| 1—(7) ,, | 40 | 30 | 80 | 2 | ◎ | 9.5 |
| 1—(8) ,, | 50 | 0 | 95 | 1 | O | 8 |
| 1—(9) ,, | 50 | 30 | 80 | 1 | ◎ | 10 |
| 1—(10) ,, | 50 | 50 | 95 | 1 | ◎ | 10 |
| 1—(11) ,, | 60 | 0 | 80 | 1 | O | 8.5 |
| 1—(12) ,, | 60 | 30 | 80 | 1 | ◎ | 11 |
| 1—(13) ,, | 60 | 30 | 70 | 1 | ◎ | 11 |
| 1—(14) ,, | 80 | 50 | 80 | 1 | ◎ | 5.5 |
| 1—(15) Control Run | 90 | 60 | 80 | 1 | ◎ | 4 |

* Expressed as percentages based on the weight of the aqueous calcium chloride solution, exclusive of ethyl alcohol.
** Expressed as percentages based on the weight of the aqueous calcium chloride solution.
*** Rated as insoluble (X), sparingly soluble (Δ), soluble (O), or very soluble ◎

### Example 2

The procedure of Test Run 1-(12) in Example 1 was repeated except that the calcium chloride was replaced by calcium nitrate. After dialysis, the resulting aqueous fibroin solution had a fibroin concentration of 6.2% by weight and a residual calcium nitrate concentration of 6.2% by weight and a residual calcium nitrate concentration of 0.015% by weight. This aqueous fibroin solution was either concentrated or diluted with water to prepare a series of aqueous fibroin solutions having the respective fibroin concentrations indicated in Table 2.

An alcohol was added to each of the above aqueous fibroin solutions and the form of the resulting gel was observed. When the fibroin concentration was less than 3% by weight at in Control Runs 2-(1) and 2-(2) or when the amount of alcohol added was greater than 150% by weight of the aqueous fibroin solution as in Control Runs 2-(6), 2-(14) and 2-(18), not a homogeneous mass of gell but a white precipitate was formed. This precipitate was too sticky to be separated by conventional filtration under reduced pressure. When it was placed in a cloth bag and then centrifuged, it aggregated into a bulky and sticky mass which was very difficult of dehydration and drying. On the other hand, when the amount of alcohol added was less than 1% by weight as in Control Runs 2-(8) and 2-(15), no gelation was recognized even after the mixture was allowed to stand for a whole day and night. Furthermore, when the fibroin concentration was greater than 20% by weight as in Control Run 2-(22), the resulting mass of gel was so tough that it could hardly be dehydrated by centrifugation.

However, the process of this invention allowed a homogeneous mass of gel to be formed in an instant or within several hours after the addition of an alcohol. When dehydrated by centrifugation, the mass of gel was broken to fragments having sizes of the order of several millimeters and water contents of 110—480% by weight. Methyl alcohol, ethyl alcohol, and isopropyl alcohol made no substantial differences in the form of the resulting gel and the requirements for further treatment. The dehydrated gel was dried in a hot-air oven set at 90—100°C or a vacuum dryer set at 70°C to obtain a fine granular product of fibroin suitable for direct pulverization with a jet mill. Subsequently, the granular product was subjected to a wet heat treatment comprising exposure to saturated steam at 120°C for 20 minutes, and then pulverized with a jet mill to obtain a fine powder of fibroin consisting of nearly globular particles, 98% or more of which had diameters of 5—40$\mu$.

When measurements of the bulk density were made, all the fine powders of fibroin, except that produced in Control Run 2-(22), were found to have values within the range of 0.1—0.7 g/cm$^3$. The reason for this seems to be that the fibroin particles contained therein had minute pores owing to the special manner of production in which a powder is derived from a homogeneous mass of gel.

In all the fine powders of fibroin produced in accordance with this invention, the content of hot-water-insoluble fibroin or rate of $\beta$-configuration was found to be not less than 70% by weight and the degree of molecular orientation was found to be not greater than one-half that of natural silk.

Furthermore, measurements of the rate of moisture absorption were made. More specifically, a sample of each fine powder of fibroin was allowed to stand in an atmosphere having a temperature of 20°C and a relative humidity of 65%, and the amount of water absorbed was measured after 2 hours. For purposes of comparison, a fine powder of fibroin produced by finely dividing silk yarn with a pulverizer had a rate of moisture absorption of 2—3% by weight as measured by the same method.

The results of these measurements are given in Table 2.

**0011 161**

TABLE 2—a

| | Fibroin Concentration (wt. %) | Addition of Alcohol | | Gelation Time (min) | Form of Gel |
|---|---|---|---|---|---|
| | | Type | Amount (wt. %) | | |
| 2—(1) Control Run | 2 | E | 10 | — | Precipitate |
| 2—(2) ,, | 2 | E | 50 | — | ,, |
| 2—(3) Test Run | 3 | E | 10 | 180 | Soft mass |
| 2—(4) ,, | 5 | E | 10 | 180 | ,, |
| 2—(5) ,, | 5 | E | 50 | 5 | ,, |
| 2—(6) Control Run | 5 | E | 180 | — | Precipitate |
| 2—(7) Test Run | 5 | M | 20 | 20 | Soft mass |
| 2—(8) Control Run | 10 | E | 0.5 | 1,440 | No gelation |
| 2—(9) Test Run | 10 | E | 2 | 300 | Somewhat hard mass |
| 2—(10) ,, | 10 | E | 10 | 180 | ,, |
| 2—(11) ,, | 10 | E | 20 | 20 | ,, |
| 2—(12) ,, | 10 | E | 50 | 5 | ,, |
| 2—(13) ,, | 10 | E | 150 | 1 | Soft mass |
| 2—(14) Control Run | 10 | E | 200 | — | Precipitate |
| 2—(15) ,, | 10 | M | 0.3 | — | No gelation |
| 2—(16) Test Run | 10 | M | 20 | 20 | Somewhat hard mass |
| 2—(17) ,, | 10 | P | 20 | 20 | ,, |
| 2—(18) Control Run | 10 | P | 200 | — | Precipitate |
| 2—(19) Test Run | 15 | E | 20 | 15 | Somewhat hard mass |
| 2—(20) ,, | 15 | M | 20 | 15 | ,, |
| 2—(21) ,, | 20 | E | 20 | 15 | ,, |
| 2—(22) Control Run | 25 | E | 20 | 15 | Hard mass |

\* E stands for ethyl alcohol. M for methyl alcohol, and P for isopropyl alcohol.

TABLE 2—b

| | Dehydration by Centrifugation | | Bulk Density (g /cm³) | Rate of Moisture Absorption (wt. %) | Content of Hot-Water-insoluble Fibroin (%) | Degree of Crystallinity (%) |
|---|---|---|---|---|---|---|
| | Water Content** (wt. %) | Degree of Dehydration*** (wt. %) | | | | |
| 2—(1) Control Run | — | — | — | — | 2 | 0 |
| 2—(2) ,, | — | — | — | — | 7 | 0 |
| 2—(3) Test Run | 480 | 87 | 0.59 | 4.2 | 76 | 43 |
| 2—(4) ,, | 450 | 79 | 0.44 | 5.5 | 79 | 46 |
| 2—(5) ,, | 320 | 96 | 0.27 | 6.1 | 98 | 68 |
| 2—(6) Control Run | — | — | — | — | 10 | 1 |
| 2—(7) Test Run | 400 | 83 | 0.41 | 5.7 | 93 | 62 |
| 2—(8) Control Run | — | — | — | — | — | — |
| 2—(9) Test Run | 410 | 55 | 0.54 | 4.5 | 74 | 43 |
| 2—(10) ,, | 270 | 73 | 0.39 | 5.3 | 88 | 48 |
| 2—(11) ,, | 150 | 86 | 0.44 | 5.1 | 90 | 60 |
| 2—(12) ,, | 110 | 92 | 0.21 | 6.2 | 98 | 65 |
| 2—(13) ,, | 180 | 92 | 0.50 | 4.8 | 83 | 42 |
| 2—(14) Control Run | — | — | — | — | 17 | 3 |
| 2—(15) ,, | — | — | — | — | — | — |
| 2—(16) Test Run | 200 | 82 | 0.45 | 5.2 | 85 | 48 |
| 2—(17) ,, | 210 | 81 | 0.45 | 5.1 | 77 | 43 |
| 2—(18) Control Run | — | — | — | — | 30 | 7 |
| 2—(19) Test Run | 200 | 71 | 0.41 | 5.0 | 81 | 48 |
| 2—(20) ,, | 190 | 73 | 0.42 | 5.0 | 81 | 46 |
| 2—(21) ,, | 260 | 48 | 0.62 | 3.7 | 70 | 41 |
| 2—(22) Control Run | — | — | 0.76 | 2.9 | 38 | 12 |

** Defined as the solvent content of the gel.

*** Defined as (the amount of solvent removed by centrifugation) / (the amount of solvent used during gelation) × 100.

Example 3

One kg of spun silk waste or bourette was immersed in 30l of water containing 0.5% by weight of marseille soap, stirred at 100°C for 1 hour to remove the sericin and oily matter almost completely, washed thoroughly with water, and then dried at 70°C. In the kneader having a capacity of 50 l, a solution of 7.5 kg of calcium chloride in 5 kg of water and 4 kg of ethyl alcohol was prepared, and 3 kg of bourette degummed as above was dissolved therein by stirring at 80°C for 1 hour. After completion of the dissolution, 9 kg of water preheated to 80°C was added. The resulting fibroin solution was filtered to remove any insoluble foreign matter such as chrysalis refuse, and then desalted by passing it through the same dialyzer of the hollow-fiber type as used in Example 1. The aqueous fibroin solution thus obtained had a fibroin concentration of 6.2% by weight and a residual calcium chloride concentration of 0.021% by weight.

When 10 kg of ethyl alcohol was added, with stirring, to 40 kg of an aqueous fibroin solution prepared as above and the resulting mixture was allowed to stand, a homogeneous mass of gel was formed in 20 minutes. This mass of gell was placed in a bag made of polyester fabric and then centrifuged to obtain fragments of gel having water contents of 250—350% by weight. After the dehydrated gel was dried in a vacuum dryer set at 80°C, a part of the resulting granular product was directly pulverized with a jet mill and the rest was subjected to a wet heat treatment comprising exposure to saturated steam at 110°C for 10 minutes and then pulverized with a jet mill. Thus, a variety of fine powders of fibroin having the respective particle diameter ranges indicated in Table 3 were obtained.

Using a mixer, each of the above fine powders of fibroin was blended with an equal weight of a commercially available fine powder or talc, and the compatibility and dispersibility of the resulting blend were examined under the microscope. It was found that its dispersibility became poor when the diameters of fibroin particles were excessively large or small. Especially when the fine powder of fibroin had particle diameters of less than $1\mu$ as in Control

Run 3-(1) or when it had particle diameters of greater than $100\mu$ as in Control Runs 3-(12) and 3-(13), the fibroin and the talc tended to aggregate separately in the form of spots. When the diameters of fibroin particles were within the range of $1$—$100\mu$ and particularly $4$—$80\mu$ as taught by this invention, the fibroin and the talc showed very good compatibility and dispersbility. In addition, a sample of each fine powder of fibroin was placed on a hand and rubbed with fingers. As a result, the fine powders of fibroin produced in accordance with this invention spread uniformly over the skin without any appreciable degree of agglomeration and showed good slip properties. Especially the fine powders of fibroin subjected to a wet heat treatment were superior in slip and non-agglomeration properties to those subjected to no wet heat treatment. This is presumed to be due to the enhancement of crystallinity of fibroin caused by the wet heat treatment. In fact, when the fine powders of fibroin produced in Test Runs 3-(4) and 3-(5) were examined by X-ray diffraction analysis, the degree of crystallinity was 28% in the fine powder of fibroin subjected to no wet heat treatment and 45% in that subjected to a wet heat treatment. Moreover, the content of hot-water-insoluble fibroin or rate of $\beta$-configuration was 52% by weight in the fine powder of fibroin subjected to no wet treatment and 98% by weight in that subjected to a wet heat treatment. However, the fine powder of fibroin produced in Control Run 3-(1), which had particle diameters of less than $1\mu$, formed round agglomerates when placed on the skin and rubbed with fingers. On the other hand, the fine powders of fibroin produced in Control Runs 3-(12) and 3-(13), which had particle diameters of greater than $100\mu$, felt rough and showed very poor slip properties.

TABLE 3

| | Particle Diameter Range ($\mu$) | Wet Heat Treatment at 110°C for 10 min | Bulk Density (g/cm³) | Dispersibility with Talc * | Slip on the Skin * |
|---|---|---|---|---|---|
| 3—(1) Control Run | <1 | Done | 0.16 | X | X (Agglomeration) |
| 3—(2) Test Run | 1—10 | ,, | 0.22 | Δ | Δ |
| 3—(3) ,, | 4—25 | ,, | 0.29 | ◎ | ◎ |
| 3—(4) ,, | 5—30 | ,, | 0.44 | ◎ | ◎ |
| 3—(5) ,, | 5—30 | Not done | 0.37 | ◎ | ◎ |
| 3—(6) ,, | 10—50 | Done | 0.51 | ◎ | ◎ |
| 3—(7) ,, | 10—50 | Not done | 0.48 | O | O |
| 3—(8) ,, | 25—80 | Done | 0.55 | ◎ | O |
| 3—(9) ,, | 5—100 | ,, | 0.53 | O | Δ |
| 3—(10) ,, | 5—100 | Not done | 0.50 | Δ | Δ |
| 3—(11) ,, | 50—100 | Done | 0.64 | Δ | Δ |
| 3—(12) Control Run | 70—150 | ,, | 0.78 | X | X |
| 3—(13) ,, | >100 | ,, | 0.83 | X | X |

\* Rated as very good (◎), good ( O ), inadequate ( Δ ), or poor ( X ).

## Example 4

In this example, spun silk waste was used as the starting material for the production of fine powders of fibroin. One kg of spun silk waste was immersed in a solution of 300 g of marseille soap in 30 l of water, stirred at 95—98°C for 3 hours to reduce its gum content to 0.1% by weight or less, washed with water, and then dried in hot air at 80°C. Anhydrous zinc chloride (ZnCl₂) was dissolved in water to prepare its aqueous solutions having the respective zinc chloride concentration indicated in Table 4, which were then heated to 70°C. By stirring in a kneader, various amounts of spun silk waste degummed as above were dissolved in 10 kg each of the zinc chloride solutions to prepare fibroin solutions having the respective fibroin concentrations indicated in Table 1. As can be seen from the data of Table 4, the process of this invention allowed the waste to be dissolved easily. However, when the zinc chloride concentration was less than 5% by weight as in Run 4-(1), it was hardly dissolved even after a long period of time (24 hours or more).

Each of the resulting fibroin solutions was mixed with a concentrated solution containing the same amount of ammonium sulfate as that of zinc chloride used, so that a gel-like precipitate of fibroin was formed. This precipitate was allowed to stand at 70°C for 20 minutes in the concentrated solution of ammonium sulfate, separated by filtration, washed with water, and then dehydrated by centrifugation. As can be seen from the data of Table 4, the process of this invention caused a homogeneous mass of gel to be formed in an instant or within several hours and, when dehydrated by centrifugation, the mass of gel was broken to fragments having sizes of the order of several millimeters. However,. when the fibroin concentration was less than 3% by weight as in Runs 4-(5) and 4-(11) or when it was greater than 20% by weight as in Runs 4-(10) and 4-(16), not a homogeneous mass of gel but a white precipitate was formed. This precipitate was too sticky to be separated by conventional filtration under reduced pressure. Then it was placed in a cloth bag and then centrifuged, it aggregated into a bulky and sticky mass which was very difficult of dehydration and drying. In each test run, the dehydrated gel was dried in a hot-air oven set at 90—100°C or a vacuum dryer set at 70°C to obtain a fine granular product of silk fibroin suitable for direct pulverization. Subsequently, the granular product was pulverized with a jet mill to obtain a fine powder of fibroin consisting of nearly globular particles, 98% or more of which had diameters of 5—40$\mu$.

When measurements of the bulk density were made, all the fine powders of fibroin produced in accordance with this invention, and not those produced in the control runs, were found to have values within the range of 0.1—0.7 g/cm³. The reason for this seems to be that the particles had minute pores owing to the special manner of production in which a powder is derived from a homogeneous mass of gel.

When measurements were made of the content of hot-water-insoluble fibroin or rate of $\beta$-configuration, all the fine powders produced in accordance with this invention were found to have values of not less than 50% by weight. In addition, X-ray diffraction analysis revealed that the degree of crystallinity was not less than 25% in all the fine powders of fibroin produced in accordance with this invention.

TABLE 4

| | Zinc Chloride Concentration (wt. %) | Fibroin Concentration (wt. %) | Solubility * | Form of Gel | Rate of $\beta$-Configuration (%) | Bulk Density (g/cm³) |
|---|---|---|---|---|---|---|
| 4—(1) Control Run | 3 | 3 | X | — | — | — |
| 4—(2) Test Run | 10 | 3 | O | Soft mass | 55 | 0.59 |
| 4—(3) ,, | 10 | 6 | O | ,, | 58 | 0.65 |
| 4—(4) ,, | 10 | 10 | O | ,, | 52 | 0.68 |
| 4—(5) Control Run | 20 | 1 | ◎ | Sticky precipitate | 20 | — |
| 4—(6) Test Run | 20 | 10 | ◎ | Soft mass | 63 | 0.60 |
| 4—(7) ,, | 20 | 20 | O | ,, | 60 | 0.65 |
| 4—(8) ,, | 40 | 10 | ◎ | ,, | 68 | 0.58 |
| 4—(9) ,, | 40 | 20 | ◎ | ,, | 64 | 0.60 |
| 4—(10) Control Run | 40 | 25 | Δ | Sticky precipitate | 5 | — |
| 4—(11) ,, | 60 | 1 | ◎ | ,, | 0 | — |
| 4—(12) Test Run | 60 | 10 | ◎ | Soft mass | 68 | 0.53 |
| 4—(13) ,, | 60 | 20 | ◎ | ,, | 63 | 0.60 |
| 4—(14) ,, | 80 | 10 | ◎ | ,, | 56 | 0.66 |
| 4—(15) ,, | 80 | 20 | ◎ | ,, | 51 | 0.68 |
| 4—(16) Control Run | 80 | 25 | O | Sticky precipitate | 5 | — |
| 4—(17) ,, | 90 | 10 | ◎ | Somewhat sticky precipitate | 44 | 0.85 |

\* Rated as insoluble (X) when the spun silk waste was not dissolved even after 24 hours, sparingly soluble (Δ) when it was dissolved in 1—2 hours, soluble (O) when it was dissolved in 0.5 — 1 hour, or very soluble (◎) when it was dissolved within 0.5 hour.

### Example 5

Spun silk waste was degummed in the same manner as described in Example 4 and used as the starting material for the production of fine powders of fibroin. Calcium chloride ($CaCl_2 \cdot 4H_2O$) was dissolved in water to prepare its aqueous solutions having the respective calcium chloride concentrations indicated in Table 5, which were then heated to 95°C. By stirring in a kneader, 10 parts of spun silk waste degummed as above were dissolved in 100 parts each of the calcium chloride solutions to prepare a series of fibroin solutions. As can be seen from the data of Table 5, the process of this invention allowed the spun silk waste to be dissolved easily. However, when the calcium chloride concentration was less than 10% by weight as in Run 5-(1), it was hardly dissolved even after a long period of time (24 hours or more).

The resulting aqueous fibroin solutions were then desalted by passing each of them through a dialyzer of the hollow-fiber type at a rate of 1 l/hr. This dialyzer was composed of 2,000 hollow fibers of regenerated cellulose having an internal diameter of 200 $\mu$, a membrane thickness of 20 $\mu$, and a length of 500 mm, both ends of these hollow fibers being bundled and sealed without blocking up their hollow bores. In this case, the ratio of membrane surface area ($cm^2$) to priming volume ($cm^3$) had a value of 100. After completion of the dialysis, the aqueous fibroin solutions had fibroin concentrations of 3.2—6.7% by weight and residual calcium chloride concentrations of 0.007—0.003% by weight.

The molecular weight of the fibroin contained in each of the dialyzed aqueous fibroin solutions was measured by gel permeation chromatography. When the calcium chloride concentration was greater than 80% by weight as in Control Runs 5-(16) and 5-(17), the molecular weight was reduced to the order of 40,000. In the aqueous fibroin solutions prepared in accordance with this invention, however, the fibroin contained therein had a molecular weight of not less than 50,000 and showed no appreciable degree of hydrolysis.

These aqueous fibroin solutions were then adjusted, by either concentration or dilution, to the respective fibroin concentrations indicated in Table 5. Each of the resulting aqueous silk fibroin solutions was agitated at room temperature and at such a high speed as to give a shear rate of the order of 100/sec. In the course of 2—3 hours' agitation, the fibroin gradually precipitated and ultimately formed a mass of gel composed of a collection of small fragments of gel. However, when the fibroin concentration was less than 3% by weight as in Control Runs 5-(2) and 5-(9) or when it was greater than 20% by weight as in Control Runs 5-(8) and 5-(15), not a homogeneous mass of gel but a white precipitate was formed. This precipitate was too sticky to be separated by conventional filtration under reduced pressure. When it was placed in a cloth bag and then centrifuged, it aggregated into a bulky and sticky mass which was very difficult of dehydration and drying. In each run, the gel so formed was dehydrated by means of a centrifuge and then dried in hot air at 105°C. Using a jet mill, the resulting powder was pulverized and then subjected to a wet heat treatment comprising exposure to saturated steam at 120°C for 15 minutes. The fine powder of fibroin consisted of nearly globular particles, 98% or more of which had diameters of 5—40$\mu$.

When measurements of the bulk density were made, all the fine powders of fibroin produced in accordance with this invention were found to have values within the range of 0.1— 0.7 g/$cm^3$. The reason for this seems to be that the fibroin particles contained therein have minute pores owing to the special manner of production in which a powder is derived from a homogeneous mass of gel.

When measurements were made of the content of hot-water-insoluble fibroin or rate of $\beta$-configuration, all the fine powders produced in accordance with this invention were found to have values of not less than 50% by weight. In addition, X-ray diffraction analysis revealed that the degree of crystallinity was not less than 25% in all the fine powders of fibroin produced in accordance with this invention.

TABLE 5

| | Calcium Chloride Concentration (wt. %) | Solubility* | Fibroin Concentration (wt. %) | Form of Gel | Rate of $\beta$-Configuration (%) | Average Molecular Weight ($\times 10^4$) | Bulk Density (g/cm³) |
|---|---|---|---|---|---|---|---|
| 5—(1) Control Run | 3 | X | — | — | — | — | — |
| 5—(2) ,, | 10 | O | 1 | Sticky precipitate | 28 | 10.2 | — |
| 5—(3) Test Run | 10 | O | 10 | Soft mass | 72 | 10.2 | 0.48 |
| 5—(4) ,, | 10 | O | 15 | ,, | 66 | 10.2 | 0.61 |
| 5—(5) ,, | 20 | O | 5 | ,, | 83 | 9.8 | 0.40 |
| 5—(6) ,, | 20 | O | 10 | ,, | 86 | 9.8 | 0.45 |
| 5—(7) ,, | 20 | O | 20 | ,, | 80 | 9.8 | 0.47 |
| 5—(8) Control Run | 20 | O | 25 | Sticky precipitate | 21 | 9.8 | 0.96 |
| 5—(9) ,, | 40 | ◎ | 1 | ,, | 42 | 8.6 | 0.74 |
| 5—(10) Test Run | 40 | ◎ | 10 | Soft mass | 98 | 8.6 | 0.33 |
| 5—(11) ,, | 40 | ◎ | 15 | ,, | 92 | 8.6 | 0.32 |
| 5—(12) ,, | 40 | ◎ | 20 | ,, | 90 | 8.6 | 0.32 |
| 5—(13) ,, | 80 | ◎ | 10 | ,, | 75 | 8.3 | 0.63 |
| 5—(14) ,, | 80 | ◎ | 20 | ,, | 77 | 8.3 | 0.70 |
| 5—(15) Control Run | 80 | ◎ | 25 | Sticky precipitate | 36 | 8.3 | 0.88 |
| 5—(16) ,, | 90 | ◎ | 10 | ,, | 48 | 4.2 | 0.93 |
| 5—(17) ,, | 90 | ◎ | 25 | ,, | 35 | 4.2 | 0.90 |

* Rated as insoluble (X) when the spun silk waste was not dissolved even after 24 hours, sparingly soluble (Δ) when it was dissolved in 1—2 hours, soluble (O) when it was dissolved in 0.5—1hour, or very soluble (◎) when it was dissolved within 0.5 hours.

### Example 6

Spun silk waste was degummed in the same manner as described in Example 4 and used as the starting material for the production of fine powders of fibroin. Anhydrous zinc chloride $(ZnCl_2)$ was dissolved in water to prepare its aqueous solution having a zinc chloride concentration of 50% by weight, which was then heated to 70°C. According to the procedure of Example 5, spun silk waste was dissolved in the aqueous calcium chloride solution and the resulting solution was dialyzed and concentrated to prepare an aqueous fibroin solution having a fibroin concentration of 10% by weight. A 100-l portion of this aqueous fibroin solution was placed in a vessel and treated, with stirring, (1) by adding thereto 0.1 N sulfuric acid drop by drop until it was adjusted to pH 4.5 (isoelectric point) and then allowing it to stand at room temperature for 10 minutes, (2) by providing a 30 kHz ultrasonic wave generator on the inside wall of the vessel and operating it at room temperature for 1 hour, or (3) by using a pipe to feed air at a rate of 10 l/min and bubble it through the aqueos fibroin solution for 10 minutes. In every case the fibroin formed a mass of gel composed of a collection of small fregments of gel. This mass of gel was dehydrated by means of a centrifuge and then dried in hot air at 105°C. Using a jet mill, the resulting powder was pulverized to particle diameters of 5—40μ and, thereafter, subjected to wet heat treatment comprising exposure to saturated steam at 120°C for 30 minutes. The fine powder of fibroin thus obtained was tested for the content of hot-water-insoluble fibroin or rate of $\beta$-configuration (by estimation from the loss of weight on boiling in water), degree of crystallinity (by X-ray diffraction analysis), and bulk density. The results are given in Table 6.

Then, the three fine powders of fibroin produced according to the above-described procedure were evaluated with respect to several properties desired in base materials for cosmetic preparations. The results are given in Table 7. It can be seen from the data of this table that, when used in cosmetic preparations, all the fine powders of fibroin produced in Example 6 exhibited remarkably good properties.

TABLE 6

| | Content of Hot-Water-insoluble Fibroin (wt. %) | Degree of Crystallinity (%) | Bulk Density (g/cm³) |
|---|---|---|---|
| Fine powder of fibroin produced by coagulation at the isoelectric point | 93 | 63 | 0.46 |
| Fine powder of fibroin produced by exposure to ultrasonic waves | 99 | 66 | 0.49 |
| Fine powder of fibroin produced by aeration | 86 | 47 | 0.57 |

TABLE 7

| | Moisture Absorption and Retention* | Affinity for the Skin* | Slip on the Skin* | Hydrophilic- Lipophilic Balance* | Covering Power* |
|---|---|---|---|---|---|
| Fine powder of fibroin produced by coagulation at the isoelectric point | ◎ | O | O | ◎ | O |
| Fine powder of fibroin produced by exposure to ultrasonic waves | ◎ | ◎ | ◎ | O | ◎ |
| Fine powder of fibroin produced by aeration | ◎ | ◎ | O | O | O |

* Rated as very good (◎), good (O), inadequate (Δ), or poor (X).

## Example 7

Spun silk waste was degummed in the same manner as described in Example 4 and used as the starting material for the production of fine powders of fibroin. An aqueous solution containing 8% by weight of ethylenediamine and 6% by weight of cupric hydroxide was prepared, and 1 kg of spun silk waste degummed as above was dissolved in 10 kg of the cupri-ethylenediamine solution by stirring at room temperature for 5 minutes. The resulting solution was immediately adjusted to pH 6.8 with 10% acetic and then diluted with water to prepare an aqueous fibroin solution having a fibroin concentration of 10% by weight. Then, according to the procedure of Example 5, this solution was dialyzed and concentrated to prepare an aqueous fibroin solution having a fibroin concentration of 15% by weight. The resulting aqueous fibroin solution was agitated at room temperature and at such a high speed as to give a shear rate of the order of 100/sec. In the course of 2—3 hours' agitation, the fibroin was gradually precipitated and ultimately formed a mass of gel. The mass of gel was dehydrated by means of a centrifuge and then dried in hot air at 105°C. Using a jet mill, the resulting powder was pulverized to particle diameters of 5—15μ and, thereafter, subjected to a wet heat treatment comprising exposure to saturated steam at 110°C for 15 minutes. When the fine powder of fibroin thus obtained was tested, its content of hot-water-insoluble fibroin or rate of β-configuration was found to be about 83% by estimation from the loss of weight on boiling in water, and 80% by infrared spectroscopic analysis. Moreover, its degree of crystallinity was found to be about 56% by X-ray diffraction analysis and its bulk density was found to be 0.52 g/cm³. On the other hand, when a fine powder of fibroin produced in the manner except for the omission of the wet heat treatment was tested, its content of hot-water-insoluble fibroin or rate of β-configuration was found to be 53% and its degree of crystallinity was found to be about 21%.

Then, the fine powders of silk fibroin produced according to the above-described procedure were evaluated with respect to several properties desired in base materials for cosmetic preparations. The results are given in Table 8. It can be seen from the data of this table that, when used in cosmetic preparations, the fine powders of fibroin produced in accordance with this invention exhibited remarkably good properties.

TABLE 8

| | Moisture Absorption and Retention* | Affinity for the Skin* | Slip on the Skin* | Hydrophilic Lipophilic Balance* | Covering Power* |
|---|---|---|---|---|---|
| Fine powder of fibroin subjected to a wet heat treatment | ◎ | ◎ | ◎ | O | O |
| Fine powder of fibroin subjected to no wet heat treatment | ◎ | O | O | O | O |

\* Rated as very good (◎), good ( O ), inadequate ( Δ ), or poor ( X ).

Furthermore, the fine powder of fibroin produced in accordance with this invention and then subjected to a wet heat treatment, a conventional powder of fibroin in fibrous form, and a powder of fibroin having a rate of $\beta$-configuration of 10% or less (as produced by spraydrying a colloidal solution of silk fibroin) were evaluated with respect to several properties desired in base materials for cosmetic preparations. The results are given in Table 9. It can be seen from the data of this table that, when used in cosmetic preparations, the fine powder of fibroin produced in accordance with this invention exhibited remarkably good properties.

TABLE 9

| | Moisture Absorption and Retention* | Affinity for the Skin* | Slip on the Skin* | Hydrophilic-Lipophilic Balance* | Covering Power* |
|---|---|---|---|---|---|
| Fine powder of fibroin in accordance with this invention | ◎ | ◎ | ◎ | O | O |
| Powder of fibroin in fibrous form | Δ | Δ | Δ | O | X |
| Powder of fibroin having a rate of $\beta$-configuration of 10% or less | ◎ | X | X | X | X |

\* Rated as very good (◎), good ( O ), inadequate ( Δ ), or poor ( X ).

Example 8

A series of fine powders of fibroin were produced in the same manner as described in Example 5. In this example, however, aqueous fibroin solutions having fibroin concentrations of 3.3—8.5% by weight were prepared according to the respective methods indicated in Table 10 and the fibroin was precipitated from these solutions according to the respective methods also indicated in Table 10. The resulting masses of gel were dehydrated and dried, subjected to a wet heat treatment at 110°C for 30 minutes, and then pulverized to particle diameters of 1—50$\mu$ by means of a jet mill. Though slight variations were noted according to the methods of dissolution and precipitation, the fine

**0 011 161**

powders of fibroin thus obtained had contents of hot-water-insoluble fibroin ranging from 70 to 95% by weight and degrees of crystallinity ranging from 40 to 60%.

It can be seen from the data of Table 10 that, when used as a base material for cosmetic preparations, the fine powders of fibroin produced according to the various methods of dissolution exhibited remarkably good properties.

TABLE 10

| Method of Dissolution | Method of Precipitation | Moisture Absorption and Retention* | Affinity for the Skin* | Slip on the Skin* | Hydrophilic-Lipophilic Balance* | Covering Power* |
|---|---|---|---|---|---|---|
| Dissolved in Schweitzer's reagent [see JIS P8101(57)] at room temperature | Precipitated by the addition of sodium chloride to a concentration of 5% | ◎ | O | O | O | O |
| Dissolved in 70% lithium bromide at 60°C. | Precipitated by agitation at a shear rate of 200/sec | ◎ | O | ◎ | O | O |
| Dissolved in 40% calcium nitrate at 85°C | ,, | ◎ | ◎ | ◎ | ◎ | O |
| Dissolved in 60% magnesium nitrate at 100% | ,, | ◎ | ◎ | ◎ | O | O |
| Dissolved in 20% calcium thiocyanate at 60°C | Precipitated by the addition of sodium chloride to a concentration of 5% | ◎ | O | O | O | O |
| Dissolved in 50% sodium thiocyanate at 60°C | Precipitated by the addition of sodium sulfate to a concentration of 5% | ◎ | ◎ | O | O | O |
| Dissolved in 40% magnesium thiocyanate at 60°C | ,, | ◎ | O | O | O | O |
| Dissolved in 45% magnesium chloride at 60°C | Precipitated by agitation at a shear rate of 50/sec | ◎ | ◎ | O | O | O |

* Rated as very good (◎), good (O), inadequate (Δ), or poor (X).

0011161

Shortly summarized the invention comprises finely powdered high-purity fibroin and a process for producing the same. The fibroin is a fine powder of regenerated fibroin in nonfibrous and particulate form which has an average molecular weight of not less than 50,000, particle diameters of from 1 to 100$\mu$, and a bulk density of from 0.1 to 0.7 g/cm$^3$ as measured in the dry state and which contains at least 50% by weight of hot-water-insoluble fibroin having the $\beta$-configuration. The process comprises dissolving a degummed silk material in at least one solvent selected from the group consisting of an aqueous cupri-ethylenediamine solution, an aqueous ammoniacal solution of cupric hydroxide, an aqueous alkaline solution of cupric hydroxide and glycerol, an aqueous lithium bromide solution, an aqueous solution of the chloride, nitrate or thiocyanate of calcium, magnesium or zinc, and an aqueous sodium thiocyanate solution; adding a coagulating salt to the resulting aqueous fibroin solution having a fibroin concentration of from 3 to 20% by weight to coagulate and precipitate the fibroin; dehydrating and drying the gel so formed; and then pulverizing the resulting powder. Alternatively, the process comprises dissolving a degummed silk material in a solvent as defined above; dialyzing the resulting aqueous fibroin solution; subjecting the dialyzed aqueous fibroin solution having a fibroin concentration of from 3 to 20% by weight to at least one treatment for coagulating and precipitating the fibroin, the treatment being selected from the group consisting of the addition of an alcohol, the addition of a coagulating salt, aeration, coagulation at the isoelectric point, exposure to ultrasonic waves, and agitation at high shear rate; dehydrating and frying the gel so formed; and then pulverizing the resulting powder. The fine powder of fibroin thus obtained is particularly useful as an additive for cosmetic preparations.

**Claims**

1. A fine powder of regenerated fibroin in nonfibrous and particulate from which has an average molecular weight of not less than 50,000 characterized in that it has a degree of molecular orientation equal to not greater than one-half that of natural silk thread particle diameters of from 1 to 100$\mu$, and a bulk density of from 0.1 to 0.7 g/cm$^3$ as measured in the dry state and which contains at least 50% by weight of hot-water-insoluble fibroin having the $\beta$-configuration.

2. A fine powder of regenerated fibroin as claimed in claim 1, characterized in that it has a degree of crystallinity of not less than 20%.

3. A fine powder of regenerated fibroin as claimed in claim 1, characterized in that it has a degree of crystallinity of not less than 40%.

4. A fine powder of regenerated fibroin as claimed in claim 1, characterized in that it has an average molecular weight of not less than 80,000.

5. A fine powder of regenerated fibroin as claimed in claim 1, characterized in that it has a bulk density of from 0.2 to 0.6 g/cm$^3$.

6. A fine powder of regenerated fibroin as claimed in claim 1, characterized in that it contains at least 90% by weight of hot-water-insoluble fibroin having the $\beta$-configuration.

7. A process for producing a fine powder of fibroin in nonfibrous and particulate form which has an average molecular weight of not less than 50,000, a degree of molecular orientation equal to not greater than one-half that of natural silk thread, particle diameters of from 1 to 100$\mu$, and a bulk density of from 0.1 to 0.7 g/cm$^3$ as measured in the dry state and which contains at least 50% by weight of hot-water-insoluble fibroin having the $\beta$-configuration, characterized in that the process comprises the steps of dissolving a degummed silk material in at least one solvent selected from the group consisting of an aqueous cupri-ethylenediamine solution, an aqueous ammoniacal solution of cupric hydroxide, an aqueous alkaline solution of cupric hydroxide and glycerol, an aqueous lithium bromide solution, an aqueous solution of the chloride, nitrate or thiocyanate of calcium, magnesium or zinc, and an aqueous sodium thiocyanate solution; adding a coagulating salt to the resulting aqueous fibroin solution having a fibroin concentration of from 3 to 20% by weight to coagulate and precipitate the fibroin; dehydrating and drying the gel so formed; and then pulverizing the resulting powder.

8. A process for producing a fine powder of fibroin in nonfibrous and particulate form which has an average molecular weight of not less than 50,000, a degree of molecular orientation equal to not greater than one-half that of natural silk thread, particle diameters of from 1 to 100$\mu$, and a bulk density of from 0.1 to 0.7 g/cm$^3$ as measured in the dry state and which contains at least 50% by weight of hot-water-insoluble fibroin having the $\beta$-configuration, characterized in that the process comprises the steps of dissolving a degummed silk material in at least one solvent selected from the group consisting of an aqueous cupri-ethylenediamine solution, an aqueous ammoniacal solution of cupric hydroxide, an aqueous alkaline solution of cupric hydroxide and glycerol, an aqueous lithium bromide solution, an aqueous solution of the chloride nitrate or thiocyanate of calcium, magnesium of zinc, and an aqueous sodium thiocyanate solution; dialyzing the resulting aqueous fibroin solution; subjecting the dialyzed aqueous fibroin solution having a fibroin concentration of from 3 to 20% by weight to at least one treatment for coagulating and precipitating the fibroin, the treatment being selected from the group consisting of the addition of an alcohol, the addition of a coagulating salt, aeration, coagulation at the isoelectric point, exposure to ultrasonic waves, and agitation at high shear rate; dehydrating and drying the gel so formed; and then pulverizing the resulting powder.

9. A process as claimed in claim 7 or 8, characterized in that the solvent is an aqueous solution of the chloride or nitrate of calcium or magnesium.

10. A process as claimed in claim 8, characterized in that the alcohol is added to the aqueous fibroin solution in an amount of from 1 to 150% by weight based on the weight of the aqueous fibroin solution.

11. A process as claimed in claim 10, characterized in that the alcohol is methyl alcohol, ethyl alcohol, or isopropyl alcohol.

12. A process as claimed in claim 7 or 8, characterized in that the coagulating salt is ammonium sulfate, sodium, sodium sulfate, or sodium chloride.

13. A process as claimed in claim 8, characterized in that the aeration is carried out by bubbling air through the aqueous fibroin solution for a period of 1 hour or more, the air being fed at a rate of at least 0.1 l/min for each liter of the aqueous fibroin solution.

14. A process as claimed in claim 8, characterized in that the coagulation at the isoelectric point is carried out by adjusting the aqueous fibroin solution to pH 4.5 and then allowing it to stand at room temperature for a period of 10 minutes or more.

15. A process as claimed in claim 8 characterized in that the exposure to ultrasonic waves is carried out by generating ultrasonic waves having frequencies of 30 kHz or higher and exposing the aqueous fibroin solution to these ultrasonic waves for a period of 1 hour or more.

16. A process as claimed in claim 8, characterized in that the agitation is carried out at a shear rate of 50/sec or more.

17. A process as claimed in claim 7 or 8, characterized in that the gel is dehydrated by centrifugation and then dried under normal or reduced pressure.

18. A process as claimed in claim 7 or 8, characterized in that the powder resulting from the dehydration and drying step is subjected to a wet heat treatment comprising exposure to saturated steam at a temperature of 50°C or above.

19. A process as claimed in claim 7 or 8, characterized in that prior to drying, the dehydrated gel is heat-treated in an aqueous ammonium sulfate solution at a temperature of 50°C or above.

20. A process as claimed in claim 8, characterized in that the dialysis step is carried out by the use of a multilayer membrane structure or bundled hollow-fiber structure satisfying the condition expressed by

$$\frac{\text{Membrane Surface Area (cm}^2)}{\text{Printing Volume (cm}^3)} \geqq 10$$

**Patentansprüche**

1. Feines Pulver aus regeneriertem Fibroin in nicht faserförmiger und feinteiliger Form mit einem Durchschnittsmolekulargewicht von nicht weniger als 50,000, dadurch gekennzeichnet, daß es einen Grad der Molekularorientierung hat, de gleich oder nicht großer als halb so groß wie der eines natürlichen Seidenfadens ist, einen Teilchendurchmesser von 1 bis 100 μm und eine Schüttdichte von 0,1 bis 0,7 g/cm³ gemessen in trockenem Zustand aufweist, und das es nicht weniger als 50 Gew.-% an Heißwasser-unlöslichem Fibroin mit β-Konfiguration enthält.

2. Feines Pulver von regeneriertem Fibroin gemäß Anspruch 1, dadurch gekennzeichnet, daß einen Kristallinitätsgrad von nicht weniger als 20% hat.

3. Feines Pulver von regeneriertem Fibroin gemäß Anspruch 1, dadurch gekennzeichnet, daß es einen Kristallinitätsgrad von nicht weniger als 40% hat.

4. Feines Pulver von regeneriertem Fibroin gemäß Anspruch 1, dadurch gekennzeichnet, daß es ein Durchschnittsmolekulargewicht von nicht weniger als 80 000 hat.

5. Feines Pulver von regeneriertem Fibroin gemäß Anspruch 1, dadurch gekennzeichnet, daß es eine Schüttdichte von 0,2 bis 0,6 g/cm³ hat.

6. Feines Pulver von regeneriertem Fibroin gemäß Anspruch 1, dadurch gekennzeichnet, daß es wenigstens 90 Gew.-% an Heißwasser-unlöslichem Fibroin mit β-Konfiguration enthält.

7. Verfahren zur Herstellung eines feinen Pulvers aus regeneriertem Fibroin in nicht faserförmiger und feinteiliger Form mit einem Durchschnittsmolekulargewicht von nicht weniger als 50 000, das einen Grad der Molekularorientierung hat, der gleich oder nicht größerals halb so groß wie der eines natürlichen Seidenfadens ist, einen Teilchendurchmesser von 1 bis 100 μm und eine Schüttdichte von 0,1 bis 0,7 g/cm³, gemessen in trockenem Zustand aufweist, und das nicht weniger als 50 Gew.-% an Heißwasser-unlöslichem Fibroin mit β-Konfiguration enthält, dadurch gekennzeichnet, daß das Verfahren die Stuffen umfaßt:

Auflösen von entgummierten Seidenmaterial in wenigstens einem Lösungsmittel, ausgewählt aus der Gruppe bestehend aus einer wäßrigen Cupri-Ethylendiaminlösung, einer wäßrigen ammoniakalischen Lösung von Cupri-Hydroxid, einer wäßrigen alkalischen Lösung von Cupri-Hydroxid und Glyzerin, einer wäßrigen Lithiumbromidlösung, einer wäßrigen Lösung des Chlorids, Nitrats oder Thiocyanats von Kalzium, Magnesium oder Zink und einer waßrigen Natriumthiocyanatlösung;

Zugabe eines Koagulierungssalzes zu der erhaltenen wäßrigen Fibroinlösung mit einem Fibroingehalt von 3 bis 20 Gew.-%, um das Fibroin zu koagulieren und auszufällen;

Dehydratisieren und Trocknen des so gebildeten Gels und dann.

Pulverisieren des erhaltenen Pulvers.

8. Verfahren zur Herstellung eines feinen Pulvers aus regeneriertem Fibroin in nicht faserförmiger und feinteiliger Form mit einem Durchschnittsmolekulargewicht von nicht weniger als 50 000, das einen Grad der Molekularorientierung hat, der gleich oder nicht größer als halb so groß wie der eines natürlichen Seidenfadens ist, einen Teilchendurchmesser von 1 bis 100 μm und eine Schüttdichte von 0,1 bis 0,7 g/cm³, gemessen in trockenem Zustand aufweist, und das nicht weniger als 50 Gew.-% an Heißwasser-unlöslichem Fibroin mit β-Konfiguration enthält, dadurch gekennzeichnet, daß das Verfahren folgende Stufen unfaßt:

Auflösen eines entgummierten Seidenmaterials in wenigstens einem Lösungsmittel, ausgewählt aus der Gruppe bestehend aus einer wäßrigen Cupri-Ethylendiaminlösung, einer wäßrigen ammoniakalischen Lösung von Cupri-Hydroxid, einer wäßrigen alkalischen Lösung von cupri-Hydroxid und Glyzerin, einer wäßrigen Lithiumbromidlösung, einer wäßrigen Lösung des Chlorids, Nitrats oder Thiocyanats von Kalzium, Magnesium oder Zink und einer waßrigen Natriumthiocyanatlösung

Dialysieren der erhaltenen wäßrigen Fibroinlösung;

Unterwerfen der dialysierten wäßrigen Fibroinlösung mit einer Fibroinkonzentration von 3 bis 20 Gew.-%, wenigstens einer Behandlung um das Fibroin zu koagulieren und auszufällen, wobei diese Behandlung ausgewählt ist aus:

Zugabe eines Alkohols, Zugabe eines Koaguliersalzes, Belüften, Koagulieren beim isoelektrischen Punkt, einer Ultraschallwellenbehandlung und Rühren mit hohem Scherkräften;

Dehydratisieren und Trocknen des so gebildeten Gels und dann Pulverisieren des erhaltenen Pulvers.

9. Verfahren gemäß Ansprüchen 7 oder 8, dadurch gekennzeichnet, daß das Lösungsmittel eine wäßrige Lösung des Chlorids oder Nitrats von Kalzium oder magnesium ist.

10. Verfahren gemäß Anspruch 8, dadurch gekennzeichnet, daß der Alkohol zu der wäßrigen Fibroinlösung in einer Menge von 1 bis 150 Gew.-%, bezogen auf das Gewicht der wäßrigen Fibroinlösung zugegeben wird.

11. Verfahren gemäß Anspruch 10, dadurch gekennzeichnet, daß der Alkohol Methylalkohol, Ethylalkohol oder Isopropylalkohol ist.

12. Verfahren gemäß Ansprüchen 7 oder 8, dadurch gekennzeichnet, daß das Koaguliersalz Ammoniumsulfat, Natriumsulfat oder Natriumchlorid ist.

13. Verfahren gemäß Anspruch 8, dadurch gekennzeichnet, daß die Belüftung durchgeführt wird, indem man Luft durch die wäßrige Fibroinlösung während einer Stunde oder länger durchleitet, wobei die Luft in einer Menge von wenigstens 0,1 1/Min. pro Liter der wäßrigen Fibroinlösung zugeführt wird.

14. Verfahren gemäß Anspruch 8, dadurch gekennzeichnet, daß das Koagulieren beim isoelektrischen Punkt durchgeführt wird, indem man die wäßrige Fibroinlösung auf einen pH von 4,5 einstellt und dann während 10 Minuten oder mehr bei Raumtemperatur stehenläßt.

15. Verfahren gemäß Anspruch 8, dadurch gekennzeichnet, daß die ultraschallwellenbehandlung durchgeführt wird, indem man Ultraschallwellen mit Frequenzen von 30 kHz oder mehr herstellt und die wäßrige Fibroinlösung diesen Ultraschallwellen während einer Stunde oder länger aussetzt.

16. Verfahren gemäß Anspruch 8, dadurch gekennzeichnet, daß das Rühren durchgeführt wird mit einer Schergeschwindigkeit von 50/Sek. oder mehr.

17. Verfahren gemäß Ansprüchen 7 oder 8, dadurch gekennzeichnet, daß das Gel durch Zentrifugieren dehydratisiert und dann unter normalem oder vermindertem Druck getrocknet wird.

18. Verfahren gemäß Ansprüchen 7 oder 8, dadurch gekennzeichnet, daß das in der Dehydratisierungs- und Trocknungsstufe erhaltene Pulver einer feuchten Wärmebehandlung ausgesetzt wird, indem man es gesättigtem Dampf bei einer Temperatur von 50°C darüber aussetzt.

19. Verfahren gemäß Ansprüchen 7 oder 8, dadurch gekennzeichnet, daß man vor dem Trocknen das dehydratisierte Gel in einer wäßrigen Ammoniumsulfatlösung bei einer Temperatur von 50°C oder mehr wärmebehandelt.

20. Verfahren gemäß Anspruch 8, dadurch gekennzeichnet, daß die Dialysestufe durchgeführt wird unter Verwendung einer mehrschichtigen Membranstruktur oder einer gebündelten Hohlfaserstruktur, die den Bedingungen:

$$\frac{\text{Membranoberfläche (cm}^2)}{\text{Füllvolumen (cm}^3)} \geqq 10$$

**Revendications**

1. Poudre fine de fibroïne régénérée sous forme particulaire et non fibreuse, ayant un poids moléculaire moyen qui n'est pas inférieur à 50 000, caractérisée en ce qu'elle a un degré d'orientation moléculaire inférieur ou égale à la moitié de celui d'un fil de soie naturelle, des particules ayant des diamètres compris entre 1 et 100 microns, et une masse volumique apparente comprise entre 0,1 et 0,7 g/cm³, mesurée à l'état sec, la poudre contenant au moins 50% en poids de fibroïne insoluble dans l'eau chaude et ayant la configuration β.

2. Poudre selon la revendication 1, caractérisée en ce qu'elle a un degré de cristallinité qui n'est pas inférieur à 20%.

3. Poudre selon la revendication 1, caractérisée en ce qu'elle a un degré de cristallinité qui n'est pas inférieur à 40%.

4. Poudre selon la revendication 1, caractérisée en ce qu'elle a un poids moléculaire moyen qui n'est pas inférieur à 80 000.

5. Poudre selon la revendication 1, caractérisée en ce qu'elle a une masse volumique apparente comprise entre 0,2 et 0,6 g/cm³.

6. Poudre selon la revendication 1, caractérisée en ce qu'elle contient au moins 90% en poids de fibroïne insoluble dans l'eau chaude et ayant la configuration $\beta$.

7. Procédé de préparation d'une fine poudre de fibroïne régénérée sous forme particulaire et non fibreuse, ayant un poids moléculaire moyen qui n'est pas inferieur à 50 000, un degré d'orientation moléculaire inférieur ou égal à la moitié de celui d'un fil de soie naturelle, des diamètres particulaires compris entre 1 et 100 microns et une masse volumique apparente comprise entre 0,1 et 0,7 g/cm³, mesurée à l'état sec, la poudre contenant au moins 50% en poids de fibroïne insoluble dans l'eau chaude et ayant la configuration $\beta$, ledit procédé étant caractérisé en ce qu'il comprend la dissolution d'une matière dégommée à base de soie dans au moins un solvant choisi dans le groupe qui comprend une solution aqueuse de cupriéthylènediamine, une solution aqueuse ammoniacale d'hydroxyde cuivrique, une solution aqueuse alcaline d'hydroxyde cuivrique et de glycérol, une solution aqueuse de bromure de lithium, une solution aqueuse de chlorure, de nitrate ou de thiocyanate de calcium, de magnésium ou de zinc, et une solution aqueuse de thiocyanate de sodium, l'addition d'un sel coagulant à la solution aqueuse résultante ayant une concentration de fibroïne comprise entre 3 et 20% en poids afin que la fibroïne coagule et précipite, la déshydratation et le séchage du gel ainsi formé, puis la pulvérisation de la poudre résultante.

8. Procédé de préparation d'une fine poudre de fibroïne sous forme particulaire et non fibreuse, ayant un poids moléculaire moyen qui n'est pas inférieur à 50 000, un degré d'orientation moléculaire inférieur ou égal à la moitié de celui d'un fil de soie naturelle, des diamètres particulaires compris entre 1 et 100 microns, et une masse volumique apparente comprise entre 0,1 et 0,7 g/cm³, mesurée à l'état sec, la poudre contenant au moins 50% en poids de fibroïne insoluble dans l'eau chaude et ayant la configuration $\beta$,, le procédé étant caractérisé en ce qu'il comprend la dissolution d'une matière dégommée à base de soie dans au moins un solvant choisi dans le groupe qui comprend une solution aqueuse de cupri-éthylènediamine, solution aqueuse ammoniacale d'hydroxyde cuivrique, une solution aqueuse alcaline d'hydroxyde cuivrique et de glycérol, une solution aqueuse de bromure de lithium, une solution aqueuse de chlorure, de nitrate ou de thiocyanate de calcium, de magnésium ou de zinc, et une solution aqueuse de thiocyanate de sodium, la dialyse de la solution aqueuse résultante de fibroïne, le traitement de cette solution dialysée, ayant une concentration de fibroïne com-rise entre 3 et 20% en poids, par au moins une opération de coagulation et de précipitation de la fibroïne, le traitement étant choisi dans le groupe qui comprend l'addition d'un alcool, l'addition d'un sel coagulant, l'aération, la coagulation au point isoélectrique, l'exposition à des ondes ultrasonores, et l'agitation avec un gradient élevé de vitesse, la déshydratation et le séchage du gel ainsi formé, puise la pulvérisation de la poudre résultante.

9. Procédé selon l'une des revendication 7 et 8, caractérisé en ce que le solvant est une solution aqueuse de chlorure ou de nitrate de calcium ou de magnésium.

10. Procédé selon la revendication 8, caractérisé en ce que l'alcool est ajouté à la solution aqueuse de fibroïne en quantité comprise entre 1 et 150% du poids de la solution aqueuse de fibroïne.

11. Procédé selon la revendication 10, caractérisé en ce que l'alcool est choisi dans le groupe qui comprend le méthanol, l'éthanol et l'isopropanol.

12. Procédé selon l'une des revendications 7 et 8, caractérisé en ce que le sel coagulant est choisi dans le groupe qui comprend le sulfate d'ammonium, le sulfate de sodium et le chlorure de sodium.

13. Procédé selon la revendication 8, caractérisé en ce que l'aération est effectuée par barbotage d'air dans la solution aqueuse de fibroïne pendant une période d'au moins 1 heure, l'air étant transmis avec undébit d'au moins 0,1 litre/minute par litre de solution aqueuse de fibroïne.

14. Procédé selon la revendication 8, caractérisé en ce que la coagulation au point isoélectrique est effectuée par réglage du pH de la solution aqueuse de fibroïne à 4,5, puis par repos à température ambiante pendant au moins 10 minutes.

15. Procédé selon la revendication 8, caractérisé en ce que l'exposition à des ondes ultrasonores est effectuée par création d'ondes ultrasonores ayant des fréquences au moins égales à 30 kHz et par exposition de la solution aqueuse de fibroïne à ces ondes ultrasonores pendant une période d'au moins 1 heure.

16. Procédé selon la revendication 8, caractérisé en ce que l'agitation est effectuée avec un gradient de vitesse au moins égale à 50/s.

17. Procédé selon l'une des revendications 7 et 8, caractérisé en ce que le gel est déshydraté par centrifugation puis séchage à pression normale ou réduite.

18. Procédé selon l'une des revendications 7 et 8, caractérisé en ce que la poudre obtenue par déshydratation et séchage est soumise à un traitement thermique humide comprenant l'exposition à de la vapeur d'eau saturée à une température de 50°C au moins.

19. Procédé selon l'une des revendications 7 et 8 caractérisé en ce que, avant séchage, le gel déshydraté subit un traitement thermique dans une solution aqueuse de sulfate d'ammonium à une température d'au moins 50°C.

20. Procédé selon la revendication 8, caractérisé en ce que la dialyse est effectuée à l'aide d'une structure ayant une membrane multi-couche ou une structure ayant des faisceaux de fibres creuses, dans des conditions telles que la relation suivante est respectée:

$$\frac{\text{Surface de la membrane, en cm}^2}{\text{Volume d'amorçage, en cm}^3} \geqq 10$$